# NEW EUROPEAN PATENT SPECIFICATION

(11) **EP 1 286 918 B2**
(45) Date of publication and mention of the opposition decision: **19.10.2011**
(45) Mention of the grant of the patent: 29.12.2004
(21) Application number: 01958635.3
(22) Date of filing: 11.05.2001
(51) Int. Cl.: C01F 7/00

(54) **IMPROVED MECHANICAL STRENGTH OF HYDROTALCITE-BASED OXIDES**
AUF HYDROTALCIT BASIERENDE OXIDE MIT VERBESSERTEN MECHANISCHEN EIGENSCHAFTEN
OXYDES A BASE D'HYDROTALCITE PRESENTANT UNE RESISTANCE MECANIQUE AMELIOREE

(30) Priority: 18.05.2000 NO 20002543
(43) Date of publication of application: 05.03.2003
(73) Proprietor: Statoil ASA, 4035 Stavanger (NO)
(72) Inventor: RYTTER, Erling, N-7049 Trondheim (NO); RÖNNEKLEIV, Morten, N-7046 Trondheim (NO); OLSBYE, Unni, N-1187 Oslo (NO)
(74) Representative: Reitstötter - Kinzebach
(86) International application number: PCT/NO2001/000196
(87) International publication number: WO 2001/087773

(56) References cited:
- WO-A1-99/41197
- GB-A- 1 462 059
- GB-A- 2 311 790
- NO-B1- 316 440
- US-A- 4 656 156
- US-A- 5 039 645
- US-A- 5 250 279
- US-A- 5 507 980

## Description

### FIELD OF INVENTION

The present invention relates to hydrotalcites. Particularly the invention relates to calcined hydrotalcites having an enhanced mechanical strength. The invention further relates to the processes of preparing such materials. The invention also relates to the use of such materials as catalyst carriers in catalytic processes, particularly for the dehydrogenation of paraffins. Further the invention relates to processes of preparing alkenes by the use of such dehydrogenation catalysts.

### BACKGROUND OF INVENTION

Mixed M²⁺(M³⁺)O materials may be obtained by calcination of a hydrotalcite-like material (HT) of general formula:

M²⁺ ₐM³⁺ _{b}(OH)_{c}(Aⁿ⁻)_{d}*xH₂O

wherein M²⁺ is at least one divalent metal; M³⁺ is at least one trivalent metal; A is an n-valent anion, n is 1 or 2 and a and b are positive numbers, a > b.

Several methods for the preparation of hydrotalcites are known from the literature:

The most common method consists in mixing a solution containing the metal salts with a basic solution, resulting in rapid precipitation of the hydrotalcite. The two aqueous solutions may either be added slowly into a third vessel where the precipitate solution holds a constant pH¹, or the metal salt solution may be added into the basic solution at varying pH. In the latter case, the precipitate is left to crystallize in the liquid after the mixing step has been completed².

In a second method, pseudo-boehmite is slurried in water, followed by addition of an organic acid such as acetic acid. Magnesium oxide is then added, and the slurry allowed to react for some hours, thus yielding a product with hydrotalcite structure. This method is described in³.

In a third method, aluminium metal and magnesium metal are reacted in 1-hexanol, and then hydrolysed by a neutral or basic, aqueous solution, resulting in a gel-like product with a hydrotalcite structure. This method is described in ⁴.

Mixed oxides derived from hydrotalcites have found wide applications, e.g. as catalysts or catalyst carrier materials, adsorbents, ion exchange materials, etc⁵.

The mechanical strength of such materials has been the focus of several studies.
In ⁶, a material is produced by dry mixing hydrotalcite (20-80%) with activated alumina (80-20%), then optionally rehydrating the mixture and finally activating it at 5-600°C. An alternative approach consists in activating the hydrotalcite at 5-600°C prior to mixing with alumina. The crush strength of the mixed material is significantly higher than for activated hydrotalcite alone. The claims cover any adsorbent or substrate with the said composition and activation treatment.
In⁷, a hydraulic cement, containing Ca, Al and Mg or Si, is mixed with aluminium powder and a CO oxidation catalyst under dry or aqueous conditions, followed by hardening at 30-100°C. Hydrotalcite is mentioned as a possible catalyst support material under the invention, but no such examples are included.
In⁸, a spray dried hydrotalcite, prepared from AlO(OH), MgO and an organic acid, is mixed with an inorganic material (such as TiO₂, ZnO, CuCrOₓ, zeolite or celite) and
water, then shaped and dried, and optionally calcined at 400-800°C. The resulting materials have a high crush strength compared to the inorganic materials alone. In one example, the influence of the calcination temperature is investigated, and indicates a decrease in the crush strength with an increasing calcination temperature, compared to the uncalcined material.
In ⁹, a binder, consisting of an Si-Al-O (kaolin or bentonite) material, is added either before or after the precipitation of a Ni-Al-(Cr) hydrotalcite. According to the patent, both methods give materials with (quote): "....(c) excellent strength and retention of this strength in operation; (d) no loss of strength or activity or leaching in steam environments, e.g. silica or potassium leaching." No comparison is made between the two binder addition methods, nor with a material without binder.

In the present work, it has surprisingly been found that the preparation of a hydrotalcite by coprecipitation in a suspension of alumina, Al₂O₃, or an alumina precursor, such as pseudo-boehmite, AlO(OH), leads to a material which has significantly higher mechanical strength than a material where alumina is added after coprecipitation and drying of the hydrotalcite, and also compared to a material obtained by addition of an Si Al-O binder.

A procedure similar to the one used here has previously been described for a Ni Al hydrotalcite-based material¹⁰. However, the purpose of that study was to decrease the Ni content of the final material, and no reference is made to the mechanical strength of the catalyst

### DETAILED DESCRIPTION OF THE INVENTION

The present invention relates to a hydrotalcite-based material having an improved mechanical strength, said hydrotalcite having the following general formula:

M²⁺ ₐM³⁺ _{b}(OH)_{c}(Aⁿ)_{d}*xH₂O

wherein M²⁺ is at least one divalent metal; M³⁺ is at least one trivalent metal; A is an n-valent anion, n is 1 or 2 and a and b are positive numbers, a > b, which hydrotalcite is deposited on alumina or an alumina precursor by coprecipitation in a liquid suspension of alumina or an alumina precursor.

In the above mentioned material M²⁺ is Mg and M³⁺ is Al.

Preferably the above mentioned liquid is water.

The hydrotalcite is prepared by a coprecipitation method. Particularly said hydrotalcite is subsequently dried and calcined at a temperature in the range 400-1300°C, preferably at 500-1000°C.

Still more preferred the calcination preferably takes place at a temperature in the range 600-900°C.

Further the present invention relates to a process for the preparation of a hydrotalcite as defined above, wherein the hydrotalcite is coprecipitated in an intimate contact with alumina or an alumina precursor in a liquid suspension.

In this process, M²⁺ is Mg and M³⁺ is Al.

In said process the alumina or alumina precursor is preferably added as a liquid suspension.

In said process said hydrotalcite is particularly prepared in a liquid suspension of alumina or an alumina precursor.

Preferably the hydrotalcite preparation takes place during simultaneous addition of a suspension of alumina or an alumina precursor.

Particularly the above mentioned liquid is water.

In the process of the invention said hydrotalcite is prepared by a coprecipitation method.

According to a preferred embodiment of this process said hydrotalcite is subsequently dried and calcined at a temperature in the range 400-1300°C, preferably at 500-1000°C.

Further according to a preferred embodiment of this process, the calcination takes place at a temperature in the range 600-900°C.

Still another aspect of the present invention involves the use of the hydrotalcite-based material defined and prepared above as a catalyst support material.

Yet a further aspect of this invention comprises a catalyst for use in the dehydrogenation of alkanes, said catalyst comprising a catalytic active metal being impregnated on the hydrotalcite-based material defined and prepared as stated above.

In this catalyst the catalytic active metal is particularly Pt.

Particularly the catalytic active metal Pt is coimpregnated with Sn.

Further the present invention also relates to a process for the catalytic dehydrogenation of propane, wherein propane is contacted with the catalyst defined above at the standard pressure, temperature and space velocity conditions of such dehydrogenation reactions.

At last the present invention relates to the use of the catalyst defined above in the dehydrogenation of propane.

### EXAMPLES

The invention is illustrated through the following examples, which must not be construed as limitations to the invention.

### General

### Characterisation

X-Ray powder diffraction was performed using Cu K_{α} radiation with a Siemens D5000 2-theta diffractometer. The BET surface area was measured using a Quantachrome monosorb apparatus. Side crushing strength measurements (SCS) were performed on a Schenck Krebel RM100 universal material test apparatus.

### Pelleting procedure

The powder (ca. 1 g) was pressed in an IR tablet press with diameter 13 mm, using a pressure of 120 kg/cm², yielding a pellet height of 5 mm.

### Example 1. Preparation of hydrotalcite on suspended pseudo-boehmite, and a catalyst supported thereon.

*ACAT-1439.* Pseudo-boehmite (AlO(OH), Vista B, 22.98 g, 0.38 mol) was suspended in distilled water (200 ml) and heated to 60°C. Two solutions were prepared; one with Mg(NO₃)₂.6H₂O (233 g, 0.91 mol) and Al(NO₃)₃.9H₂O (34.0 g, 0.09 mol) in distilled water (900 ml), and another with Na₂CO₃ (4.8 g, 0.045 mol) and NaOH (45.2 g, 1.1 mol) in distilled water (900 ml). The two solutions were dripped into the aqueous suspension of pseudo-boehmite (duration 45 min). The pH in the precipitate solution was 9.5-10. The precipitate was filtered, then washed to neutrality and left overnight.

SnCl₂2H₂O (0.4804 g, 2.13 mmol) was dissolved in conc. HCl (10 ml). H₂PtCl₆·6H₂O (0.157 g, 0.3 mmol) was dissolved in distilled water (50 ml), and the tin chloride solution added. The resulting solution had a red colour.

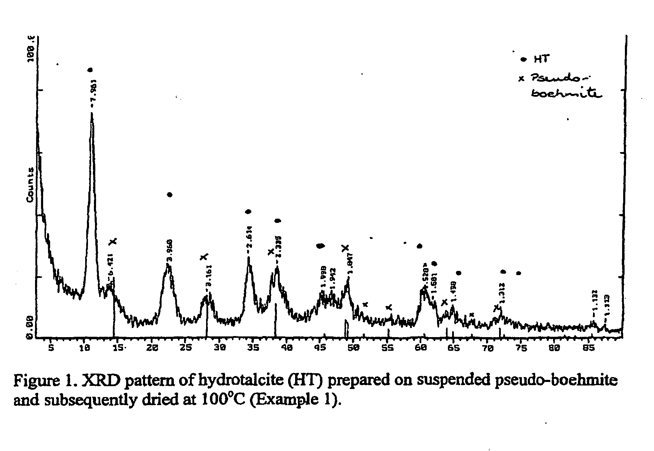

The Mg-Al precipitate was suspended in distilled water (400 ml) and the Pt-Sn solution dripped into the suspension, which had a neutral pH value. The suspension was stirred for 45 min., then filtered and washed twice with distilled water. The product was then dried at 100°C/16 h and subjected to XRD measurements. The XRD pattern clearly showed the presence of both hydrotalcite and pseudo-boehmite (Figure 1). Calcination of the product was performed at 800°C/15 hours, yielding a BET area of 118 m²/g. After pelletisation, the BET area dropped to 103 m²/g.

*ACAT-1440.* Theta-alumina (Puralox Nwa-85, 23.0 g, 0.23 mol) was suspended in distilled water (200 ml) and heated to 60°C. Two solutions were prepared; one with Mg(NO₃)_{2·}6H₂O (233 g, 0.91 mol) and Al(NO₃)_{3·}9H₂O (34.0 g, 0.09 mol) in distilled water (900 ml), and another with Na₂CO₃ (4.8 g, 0.045 mol) and NaOH (45.2 g, 1.1 mol) in distilled water (900 ml). The two solutions were dripped into the aqueous suspension of theta-alumina (duration 45 min). The pH in the precipitate solution was 9.5-10. The precipitate was filtered, then washed to neutrality and left overnight.

SnCl_{2·}2H₂O (0.4804 g, 2.13 mmol) was dissolved in conc. HCl (10 ml). H₂PtCl_{6·}6H₂O (0.158 g, 0.38 mmol) was dissolved in distilled water (50 ml), and the tin chloride solution added. The resulting solution had a red colour.

The precipitate was suspended in distilled water (400 ml) and the Pt-Sn solution dripped into the suspension, which had a neutral pH value. The suspension was stirred for 45 min., then filtered and washed twice with distilled water. The product was then dried at 100°C/16 h. Calcination of the product was performed at 800°C/15 hours, yielding a BET area of 91 m²/g. After pelletisation, the BET area dropped to 77 m²/g.

The X-Ray diffraction pattern of the final material indicated a strong interaction between the suspended alumina and the solution: When the alumina particles were crushed and sieved to a particle size < 90 micron prior to suspension, then only the hydrotalcite phase (and no alumina phase) was visible by XRD in the final catalyst. With larger or mixed particle sizes, both the hydrotalcite and alumina phases were visible by XRD after the precipitation step. The catalyst used in the further study (Example 5 below) is the one with small alumina particles (<90 micron).

### Example 3. Comparative Example A.

*C440-104.* An Mg-Al hydrotalcite was prepared according to Example 1, but without a suspended binder material. After precipitation, the material was impregnated with Pt and Sn, then dried and calcined at 800°C/15 hours. The BET area of the calcined product was 145 m²/g.

### Example 4. Comparative Example B.

*ACAT 1443.* A material was prepared according to a procedure described in GB 2 311 790 (to British Gas), but with some modifications in the precipitate composition (e.g. Mg was used as a cation instead of Ni).

### Three aqueous mixtures were prepared:

Solution A: Mg(NO₃)_{2·}6H₂O (116.5 g, 0.45 mol) and Al(NO₃)_{3·}9H₂O (17.0 g, 0.045 mol) in distilled water (500 ml).
Solution B: Na₂CO₃ (78.4 g, 0.74 mol) in distilled water (500 ml).
Suspension C: Kaolin (2.62 g) and MgO (1.24 g, 0.03 mol) in distilled water (30 ml).

Solutions A and B were heated to 75°C. Solution B was dripped into solution A under stirring (duration: 30 min). The pH in the precipitate solution was 10. Suspension C was added and the final mixture stirred for some minutes. The product was filtered and then washed several times with distilled water.

SnCl_{2·}2H₂O (0.240 g, 1.06 mmol) was dissolved in conc. HCl (10 ml). H₂PtCl_{6·}6H₂O (0.078 g, 0.19 mmol) was dissolved in distilled water (50 ml), and the tin chloride solution added. The resulting solution had a red colour.

The precipitate was suspended in distilled water (200 ml) and the Pt-Sn solution dripped into the suspension, which had a neutral pH value. The suspension was stirred for 45 min., then filtered and washed twice with distilled water. The product was then dried at 100°C/16 h and calcined at 450°C/5 hours. The product was then crushed and dry mixed with Secar 71 (Lafarge, 7.2 g) (a mixture of CaO and Alumina) and 2wt% graphite, and then stirred for 1 h. The product was pelleted, steamed at 240°C/16h and soaked in distilled water (16 h). The soaking procedure led to pellet cracking. Subsequently, the pellets were dipped in a 2% KOH aqueous solution. The pellets were crushed and the material pelleted once more. XRD of the product showed the presence of a crystalline hydrotalcite phase, as well as MgO and Secar-71 (Ca₃Al₁₀O₁₈). The BET area of the product was 30 m²/g, which is within the expected range for uncalcined hydrotalcites. XRD of the same product after use as a PDH catalyst (Example 5) indicated that the hydrotalcite phase is converted to Mg(Al)O during use as a PDH catalyst.

### Example 5. Propane dehydrogenation (PDH) tests

The materials prepared according to Examples 1-4 were subjected to testing under PDH conditions at 600°C in a quartz reactor with i.d. 23 mm. The reactor was heated to 600°C in a N₂ flow, then subjected to reduction, PDH and regeneration test cycles according to Table 1. The GHSV was 1000 h⁻¹ based on propane. The test was stopped after 6 test cycles with regeneration, and the catalyst cooled to room temperature in a N₂ flow. GC analysis of the reactor effluent showed the presence of both propane and propene from all catalysts.

**Table 1.**

| PDH test conditions. | | | | | | |
|---|---|---|---|---|---|---|
| | Step 1 Reduction | Step 2 PDH | Step 3 1%O₂ | Step 4 5% O₂ | Step 5 10% O₂ | Step 6 20%O₂ |
| N₂ (ml/min) | 241 | | 277 | 218 | 146 | |
| Air (ml/min) | | | 14.3 | 73 | 146 | 291 |
| Propane (ml/min) | | 92.9 | | | | |
| H₂O (g/h) | | 8.3 | | | | |
| H₂ (ml/min) | 50 | 13.1 | | | | |
| Duration (min) | 30 | 1200 | 60 | 60 | 60 | 60 |

Visual inspection of the tested pellets showed no sign of damage for the samples prepared according to Examples 1,2 and 4. However, some fine powder had been formed from the sample prepared without binder (Example 3) during testing. Judging from the remaining pellets, this powder originated from the edges of each pellet. None of the pellets were cracked.

### Example 6. Mechanical strength of pellets

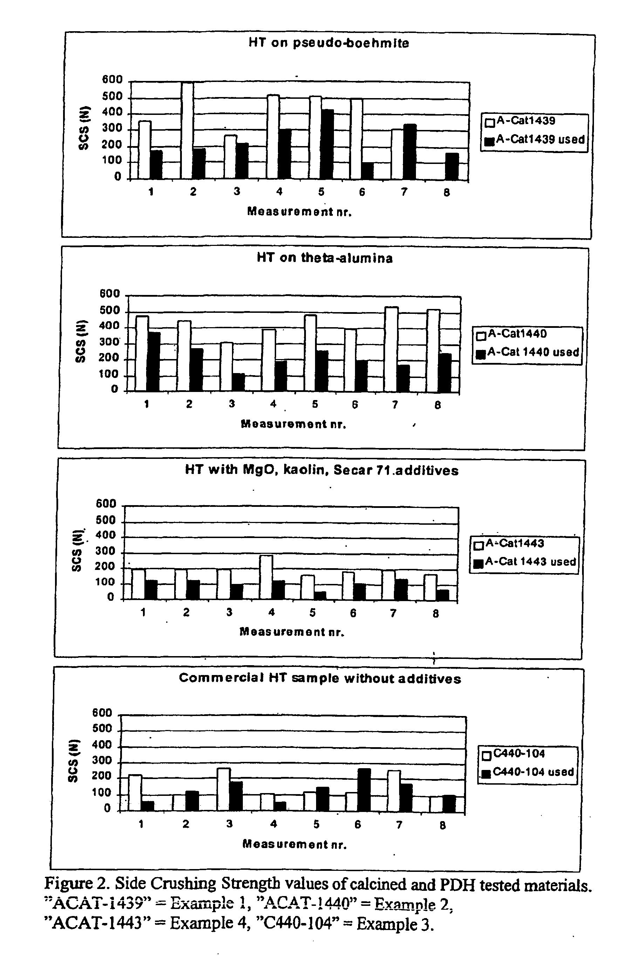

The materials which had been prepared according to Examples 1-4, and tested according to Example 5, were subjected to mechanical strength measurements by using a Side Crushing Strength procedure (SCS). The SCS raw data for each material are shown in Figure 2. As can be seen, the mechanical strength measured for different pellets of one catalyst varies significantly. However, when calculating the average SCS value for each catalyst, as shown in Table 2, some trends are observed:
- The catalysts that are precipitated on suspended pseudo-boehmite and theta-alumina (Examples 1 and 2) have significantly higher SCS values than the sample prepared without binder (Example 3).
- Alumina and pseudo-boehmite give materials with similar mechanical strength before and after testing.
- The catalyst prepared using the modified BG recepy (Example 4) has a similar mechanical strength to the sample prepared without binder (Example 3).
- The mechanical strength decreases during PDH testing for all samples, especially for the samples with the highest initial SCS value. However, even after 1 week of testing, the SCS value of the strongest sample (Example 1) is 74% higher than for the sample prepared without binder (Example 3).
- The mechanical strength of the final material seems to be uninfluenced by whether or not the crystallinity of the alumina (precursor) is maintained throughout the precipitation and metal deposition steps.

**Table 2.**

| Average SCS values for the materials in Examples 1-4 before and after testing as PDH catalysts for 1 week. | | |
|---|---|---|
| Catalyst | SCS before testing (N) | SCS after PDH testing (N) |
| 1 (ACAT-1439) | 436 | 238 |
| 2 (ACAT-1440) | 441 | 224 |
| 3 (C440-104) | 174 | 137 |
| 4 (ACAT-1443) | 196 | 100 |

### Comparative example 7. Addition of alumina after precipitation and calcination of the hydrotalcite.

A Mg-Al catalyst was prepared according to Example 1, but without a suspended material in the precipitation vessel. After completing the precipitation and metal addition steps, the hydrotalcite material was dried at 100°C/16 hours, and then dry mixed with pseudo-boehmite-(AlO(OH), Vista B, 22.98 g, 0.38 mol). The mixture was then calcined at 800°C/15 hours, and subsequently pelleted and subjected to SCS measurements.

The average SCS value of the calcined material was ---- N. This result shows that dry mixing the calcined hydrotalcite with alumina significantly enhances the mechanical strength of the final material compared to calcined hydrotalcite alone. However, the mechanical strength of this material is clearly inferior to the mechanical strength obtained by preparing the hydrotalcite in a suspension of hydrotalcite (Example 1).

### Comparative Example 8. Alumina addition after precipitation of the hydrotalcite

A catalyst was prepared according to Example 1, but without a suspended material in the precipitation vessel. After the precipitation and metal deposition steps, but before drying, Pseudo-boehmite (AlO(OH), Vista B, 22.98 g, 0.38 mol) was added to the precipitate. The final material was subjected to drying (100°C/16 hours), calcination (800°C/15 hours), pelletisation and SCS measurements. The average SCS value of the pellets was ― N.

This result shows that adding the alumina precursor after precipitation of the hydrotalcite, but before drying, leads to a material with a similar mechanical strength to the material where the alumina precursor is added before precipitation. A comparison between Example 7 and Example 8 indicates that it is advantageous to add the alumina (precursor) before drying the hydrotalcite.

### Example 9. Preparation of hydrotalcite in a lower quantity of suspended pseudo-boehmite

A material was prepared according to Example 1, with the only exception that a lower amount of pseudo-boehmite (AlO(OH), Vista B, 4.60 g, 0.076 mol) was used. The average SCS value of the final material was―N. This result shows that a material with a high mechanical strength may be obtained by the suspension - precipitation method, even when the amount of alumina in the final material is quite low.

### Conclusion

The Examples above clearly illustrate that the presence of an alumina or alumina precursor support in a hydrotalcite-based oxide material, i. e. the hydrotalcite-based oxide material is deposited on said alumina or alumina precursor support, uexpectedly leads to an enhanced mechanical strength both initially and after PDH testing. The use of alumina itself, or of a hydrated form of alumina, give similar results. The PDH tests should be considered as a tool to illustrate that the materials can withstand quite severe conditions, i.e. cycling between coking, steam-rich conditions and oxidizing conditions, all at ca. 600°C.

Further, the Examples illustrate that the addition of the alumina (precursor) as a liquid suspension before precipitation of the hydrotalcite is particularly advantageous.

Finally, the Examples illustrate that a silica-based binder described in the literature⁵ gives only a slight improvement of the mechanical strength of the materials used here, compared to a similar material with no binder.

In conclusion, the Examples illustrate that the binder composition, as well as the method of binder addition, are both of major importance for the mechanical strength of hydrotalcite-based materials.

It is to be expected that this result is not restricted to the hydrotalcite preparation method used in the Examples, but is also achievable for other hydrotalcite preparation methods. It is further expected that the results are valid also for an uncalcined hydrotalcite-like material.

These results were not to be expected in view of the prior art known to the applicant on the filing date of the instant application.

The examples presented above must in now way be construed as a limitation of the present invention, but merely as illustrations thereof, the scope of the invention being fully defined in the appending claims.

### Literature References:

¹H, Schaper, J.J. Berg-Slot, W.H.J. Stork, Appl. Cat. 54 (1989) 79
² W.T. Reichle, J. Catal. 94 (1985) 547
³ C.P. Kelkar, A.A. Schutz, L.A. Cullo, US 5 507 980 (1995); to Aristech Chemical Corporation
⁴ K. Diblitz, K. Noweck, J. Schiefler, A. Brasch, WO 98/23727 (1996); to CONDEA
⁵ F. Cavani, F. Trifirò, A. Vaccari, Cat. Today, 11(2), (1991), 171
⁶ C. Misra, US 4,656,156 (1986); to Aluminium Company of America
⁷ D.J. Elliott, P.A. Tooley, US 5,039,645 (1990); to Phillips Petroleum Company
⁸ C.P. Kelkar, A.A. Schutz, L.A. Cullo, US 5 507 980 (1995); to Aristech Chemical Corporation
⁹ S.B.J. Scott, GB 2 311 790 (1996); to British Gas
¹⁰ UK Patent 1 462 059-60 (1973); to BASF AG

## Claims

1. A hydrotalcite-based material having an improved mechanical strength, said hydrotalcite having the following general formula:
m²⁺ₐM³⁺_{b}(OH)_{c}(Aⁿ⁻)_{d}*xH₂O
wherein M²⁺ is Mg; M³⁺ is Al; A is an n-valent anion, n is 1 or 2 and a and b are positive numbers, a > b, which hydrotalcite is deposited on alumina or an alumina precursor by coprecipitation in a liquid suspension of alumina or an alumina precursor.

2. The material according to claim 1, wherein the liquid is water.

3. The material according to claim 1 or 2, said hydrotalcite being subsequently dried and calcined at a temperature in the range 400 - 1300 °C, preferably at 500-1000 °C.

4. The material according to claim 3, wherein the calcination preferably takes place at a temperature in the range 600 - 900 °C.

5. A method for the preparation of a hydrotalcite as defined in any one of claims 1 to 4 **characterized in that** the hydrotalcite is coprecipitated in an intimate contact with alumina or an alumina precursor in a liquid suspension.

6. The method of claim 5, wherein the alumina or alumina precursor is added as a liquid suspension.

7. The method of claim 5 or 6, wherein hydrotalcite preparation takes place during simultaneous addition of a suspension of alumina or an alumina precursor.

8. The method of any one of claims 5-7, wherein the liquid is water.

9. The method of any one of claims 5-8, said hydrotalcite being subsequently dried and calcined at a temperature in the range 400 - 1300 °C, preferably at 500 - 1000 °C.

10. The method of claim 9, wherein the calcination preferably takes place at a temperature in the range 600 - 900 °C.

11. Use of a hydrotalcite-based material of any one of claims 1 - 4 as a catalyst support material.

12. A catalyst for use in the dehydrogenation of alkanes, said catalyst comprising a catalytic active metal being impregnated on the hydrotalcite-based material of any one of claims 1-4.

13. The catalyst of claim 12, wherein the catalytic active metal is Pt.

14. The catalyst of claim 13, wherein the catalytic active metal Pt is coimpregnated with Sn.

15. A process for the catalytic dehydrogenation of propane, wherein propane is contacted with the catalyst of claims 12 - 14 at the standard pressure, temperature and space velocity conditions for such dehydrogenation reactions.

## Patentansprüche

1. Material auf Hydrotalcit-Basis mit verbesserter mechanischer Festigkeit, wobei der Hydrotalcit die folgende allgemeine Formel aufweist:
M²+ₐM³+_{b}(OH)_{c}(Aⁿ⁻)d*xH₂O
worin M²⁺ für Mg steht; M³⁺ für Al steht; A für ein n-wertiges Anion steht; n für 1 oder 2 steht und a und b für positive Zahlen stehen, a > b; und der Hydrotalcit durch Kopräzipitation in einer flüssigen Suspension von Aluminiumoxid oder einem Aluminiumoxidvorläufer auf Aluminiumoxid oder einem Aluminiumoxidvorläufer abgeschieden ist.

2. Material nach Anspruch 1, wobei es sich bei der Flüssigkeit um Wasser handelt.

3. Material nach einem der Ansprüche 1 bis 2, wobei man den Hydrotalcit anschließend trocknet und bei einer Temperatur im Bereich von 400 bis 1300 °C, vorzugsweise 500 bis 1000 °C kalziniert.

4. Material nach Anspruch 3, wobei die Kalzinierung vorzugsweise bei einer Temperatur im Bereich von 600 bis 900 °C erfolgt.

5. Verfahren zur Herstellung eines Hydrotalcits gemäß Definition in einem der Ansprüche 1 bis 4, **dadurch gekennzeichnet, dass** man den Hydrotalcit in innigem Kontakt mit Aluminiumoxid oder einem Aluminiumoxidvorläufer in einer flüssigen Suspension kopräzipitiert.

6. Verfahren nach Anspruch 5, wobei man das Aluminiumoxid oder den Aluminiumoxidvorläufer in Form einer flüssigen Suspension zufügt.

7. Verfahren nach einem der Ansprüche 5 oder 6, wobei die Hydrotalcitherstellung gleichzeitig mit der Zugabe einer Suspension von Aluminiumoxid oder eines Aluminiumoxidvorläufers erfolgt.

8. Verfahren nach einem der Ansprüche 5 bis 7, wobei es sich bei Flüssigkeit um Wasser handelt.

9. Verfahren nach einem der Ansprüche 5 bis 8, wobei man den Hydrotalcit anschließend trocknet und bei einer Temperatur im Bereich von 400 bis 1300 °C, vorzugsweise 500 bis 1000 °C, kalziniert.

10. Verfahren nach Anspruch 9, wobei die Kalzinierung vorzugsweise bei einer Temperatur im Bereich von 600 bis 900 °C erfolgt.

11. Verwendung eines Materials auf Hydrotalcit-Basis nach einem der Ansprüche 1 bis 4 als Katalysatorträgermaterial.

12. Katalysator zur Verwendung zur Dehydrierung von Alkanen, wobei der Katalysator ein katalytisch aktives Metall umfasst, mit dem das Material auf Hydrotalcit-Basis nach einem der Ansprüche 1 bis 4 imprägniert ist.

13. Katalysator nach Anspruch 12, wobei es sich bei dem katalytisch aktiven Metall um Pt handelt.

14. Katalysator nach Anspruch 13, wobei es sich bei dem katalytisch aktiven Metall um mit Sn coimprägniertes Pt handelt.

15. Verfahren zur katalytischen Dehydrierung von Propan, wobei man Propan mit dem Katalysator nach den Ansprüchen 12 bis 14 bei für derartige Dehydrierungsreaktionen üblichen Druck-, Temperatur- und Raumgeschwindigkeitsbedingungen in Kontakt bringt.

## Revendications

1. Matériau à base d'hydrotalcite ayant une résistance mécanique améliorée, ladite hydrotalcite présentant la formule générale suivante :
M²⁺ₐM³⁺_{b}(OH)_{c}(Aⁿ⁻)_{d}*XH₂O
dans laquelle M²⁺ est Mg; M³⁺ est Al ; A est un anion n-valent, n est égal à 1 ou à 2 et a et b sont des nombres positifs, a > b, laquelle hydrotalcite est déposée sur de l'alumine ou un précurseur d'alumine par co-précipitation dans une suspension liquide d'alumine ou d'un précurseur d'alumine.

2. Matériau selon la revendication 1, dans lequel le liquide est l'eau.

3. Matériau selon la revendication 1 ou 2, ladite hydrotalcite étant ensuite séchée et calcinée à une température dans l'intervalle de 400-1 300°C, de préférence à 500-1 000°C.

4. Matériau selon la revendication 3, dans lequel la calcination est de préférence réalisée à une température dans l'intervalle de 600-900°C.

5. Procédé pour la préparation d'une hydrotalcite comme défini dans l'une quelconque des revendications 1 à 4, **caractérisé en ce que** l'hydrotalcite est co-précipitée en contact intime avec de l'alumine ou un précurseur d'alumine dans une suspension liquide.

6. Procédé selon la revendication 5, dans lequel l'alumine ou le précurseur d'alumine est ajouté comme une suspension liquide.

7. Procédé selon la revendication 5 ou 6, dans lequel la préparation d'hydrotalcite est réalisée pendant l'addition simultanée d'une suspension d'alumine ou d'un précurseur d'alumine.

8. Procédé selon l'une quelconque des revendications 5-7, dans lequel le liquide est l'eau.

9. Procédé selon l'une quelconque des revendications 5-8, ladite hydrotalcite étant ensuite séchée et calcinée à une température dans l'intervalle de 400-1 300°C, de préférence à 500-1 000°C.

10. Procédé selon la revendication 9, dans lequel la calcination est réalisée de préférence à une température dans l'intervalle de 600-900°C.

11. Utilisation d'un matériau à base d'hydrotalcite selon l'une quelconque des revendications 1-4 comme matériau de support de catalyseur.

12. Catalyseur destiné à être utilisé dans la déshydrogénation d'alcanes, ledit catalyseur comprenant un métal catalytique actif étant imprégné sur le matériau à base d'hydrotalcite selon l'une quelconque des revendications 1-4.

13. Catalyseur selon la revendication 12, dans lequel le métal catalytique actif est Pt.

14. Catalyseur selon la revendication 13, dans lequel le métal catalytique actif Pt est co-imprégné avec Sn.

15. Procédé pour la déshydrogénation catalytique de propane, dans lequel du propane est mis en contact avec le catalyseur des revendications 12-14 dans des conditions standards de pression, de température et de vitesse spatiale pour de telles réactions de déshydrogénation.
